Europäisches Patentamt

European Patent Office

Office européen des brevets

(19)

(11) **EP 0 582 930 B1**

(12) **EUROPÄISCHE PATENTSCHRIFT**

(45) Veröffentlichungstag und Bekanntmachung des Hinweises auf die Patenterteilung:
**03.09.1997 Patentblatt 1997/36**

(51) Int. Cl.$^6$: **C07C 68/06**, C07C 69/96

(21) Anmeldenummer: **93112353.3**

(22) Anmeldetag: **02.08.1993**

(54) **Verfahren zur Herstellung von Diarylcarbonaten**

Process for the preparation of diarylcarbonates

Procédé pour la préparation de diarylcarbonates

(84) Benannte Vertragsstaaten:
**BE DE ES FR GB IT NL**

(30) Priorität: **13.08.1992 DE 4226756**

(43) Veröffentlichungstag der Anmeldung:
**16.02.1994 Patentblatt 1994/07**

(73) Patentinhaber: **BAYER AG**
**51368 Leverkusen (DE)**

(72) Erfinder:
• **Schön, Norbert, Dr.**
**D-47800 Krefeld (DE)**

• **Buysch, Hans-Josef, Dr.**
**D-47809 Krefeld (DE)**
• **Wagner, Paul, Dr.**
**D-40597 Düsseldorf (DE)**
• **Langer, Reinhard, Dr.**
**D-47800 Krefeld (DE)**

(56) Entgegenhaltungen:
**EP-A- 0 461 274**

• **PATENT ABSTRACTS OF JAPAN vol. 11 no. 163 (C-424) [2610] ,26.Mai 1987 & JP-A-61 291545 (DAICEL CHEM IND LTD)**
• **PATENT ABSTRACTS OF JAPAN vol. 11 no. 163 (C-424) [2610] ,26.Mai 1987 & JP-A-61 291545**

Printed by Rank Xerox (UK) Business Services
2.14.14/3.4

**Beschreibung**

Die Erfindung betrifft ein kontinuierlich arbeitendes Verfahren zur Herstellung von Diarylcarbonaten oder Alkylarylcarbonaten aus Dialkylcarbonaten und Phenolen unter Verwendung von üblichen Umesterungskatalysatoren, das dadurch gekennzeichnet ist, daß zunächst die Ausgangsprodukte miteinander in der Flüssigphase im Gegenstrom umgesetzt werden und die Reaktion in einer Reaktionskolonne zu Ende geführt wird.

Die Herstellung von aromatischen und aliphatisch-aromatischen Kohlensäureestern (Carbonaten) durch Umesterung, ausgehend von aliphatischen Kohlensäureestern und Phenolen, ist im Prinzip bekannt. Dabei handelt es sich um eine Gleichgewichtsreaktion, wobei die Lage des Gleichgewichts fast vollständig in Richtung der aliphatisch substituierten Carbonate verschoben ist. Daher ist es verhältnismäßig leicht, aus aromatischen Carbonaten und Alkoholen aliphatische Carbonate herzustellen. Um die Reaktion jedoch im umgekehrten Sinne in Richtung aromatischer Carbonate durchzuführen, ist es notwendig, das sehr ungünstig liegende Gleichgewicht effektiv zu verschieben, wobei nicht nur sehr aktive Katalysatoren, sondern auch eine günstige Verfahrensweise zur Anwendung gelangen müssen.

Zur Umesterung von aliphatischen Kohlensäureestern mit Phenolen sind eine Vielzahl von effektiven Katalysatoren, wie beispielsweise Alkalihydroxide, Lewissäure-Katalysatoren aus der Gruppe der Metallhalogenide (DE-OS 25 28 412 und DE-OS 25 52 907), Organozinnverbindungen (EP 879, EP 880, DE-OS 34 45 552, EP 338 760), Bleiverbindungen (JP-57/176 932), Lewissäure/Protonensäure-Katalysatoren (DE-OS 34 45 553), empfohlen worden. In den bekannten Verfahren wird die Umesterung in einem chargenweise betriebenen Reaktor drucklos oder unter Druck, gegebenenfalls mit einer zusätzlichen Trennkolonne, durchgeführt. Dabei werden auch mit den aktivsten Katalysatoren Reaktionszeiten von vielen Stunden bis zum Erteichen auch nur mittlerer Umsätze von ungefähr 50 % an Phenol benötigt. So werden bei der chargenweise betriebenen Umesterung von Phenol mit Diethylcarbonat bei 180°C unter Verwendung verschiedener Organozinnverbindungen, wie sie in DE-OS 34 45 552 beschrieben werden, Ausbeuten an Diphenylcarbonat in einer Größenordnung von mehr als 20 % erst nach ca. 24-stündiger Reaktionszeit erreicht; bei der chargenweise betriebenen Umesterung von Phenol und Dimethylcarbonat mit Hilfe von Organozinnkatalysatoren, wie sie in EP-879 beschrieben sind, beträgt der Phenolumsatz nach 30 Stunden 34 % des theoretischen Wertes.

Das bedeutet, daß aufgrund der ungünstigen thermodynamischen Voraussetzungen die beschriebenen Umesterungsreaktionen in Kesseln oder Druckautoklaven auch bei Verwendung sehr aktiver Katalysatorsysteme im Sinne eines technischen Prozesses nur sehr unvorteilhaft durchführbar sind, da sehr schlechte Raum-Zeit-Ausbeuten und hohe Verweilzeiten bei hohen Reaktionstemperaturen erforderlich sind, wobei wegen der unvollständigen Umesterung zusätzlich ein hoher Destillationsaufwand aufgebracht werden muß, der weitere Energie erforderlich macht.

Solche Verfahrensweisen sind auch deshalb besonders unvorteilhaft, da selbst mit sehr selektiven Umesterungskatalysatoren bei den hohen Temperaturen und lagen Verweilzeiten von vielen Stunden ein merklicher Anteil an Nebenreaktionen auftritt, beispielsweise die Etherbildung und die Abspaltung von Kohlendioxid.

Es wurde daher versucht, das Reaktionsgleichgewicht durch Adsorption des bei der Umesterung entstehenden Alkohols an Molekularsieben möglichst schnell in Richtung der gewünschten Produkte zu verschieben (DE-OS 33 08 921). Aus der Beschreibung dieser Reaktion zeigt sich, daß zur Adsorption des Reaktionsalkohols eine groß Menge an Molekularsieb benötigt wird, die die Menge an freiwerdendem Alkohol weit überschreitet. Weiterhin müssen die eingesetzten Molekularsiebe schon nach kurzer Zeit regeneriert werden, und die Umwandlungsrate zu den Alkylarylcarbonat-Zwischenprodukten ist relativ gering. Auch dieses Verfahren erscheint deshalb als technisch nicht vorteilhaft anwendbar.

Es ist bekannt, Gleichgewichtsreaktionen, insbesondere Veresterungen und Umesterungen in Kolonnen durchzuführen und sie auf diese Weise vorteilhaft in Richtung Produktbildung zu verschieben (z.B. U.Block, Chem.-Ing.-Techn. **49**, 151 (1977); DE-OS 3 809 417; B. Schleper, B. Gutsche, J. Wnuck und L. Jeromin, Chem.-Ing.-Techn. **62**, 226 (1990); Ullmanns Encyclopädie der techn. Chemie, 4. Aufl., Bd. 3, S. 375 ff., 1973; ibid, 5. Aufl., Vol. B4, S. 321 ff., 1992).

In EP 0 461 274 (WO 91/09832) wird ein kontinuierlicher Umesterungsprozeß zur Herstellung von aromatischen Carbonaten in einer oder in mehreren hintereinander geschalteten mehrstufigen Kolonnen beschrieben, wobei Dialkylcarbonate oder Alkylarylcarbonate mit Phenolen umgesetzt werden und am Kopf der Kolonnen die leichtflüchtigen Produkte, nämlich Reaktionsalkohole und Dialkylcarbonate und am Fuß der Kolonnen die schwersiedenen Produkte, nämlich Arylcarbonate entnommen werden.

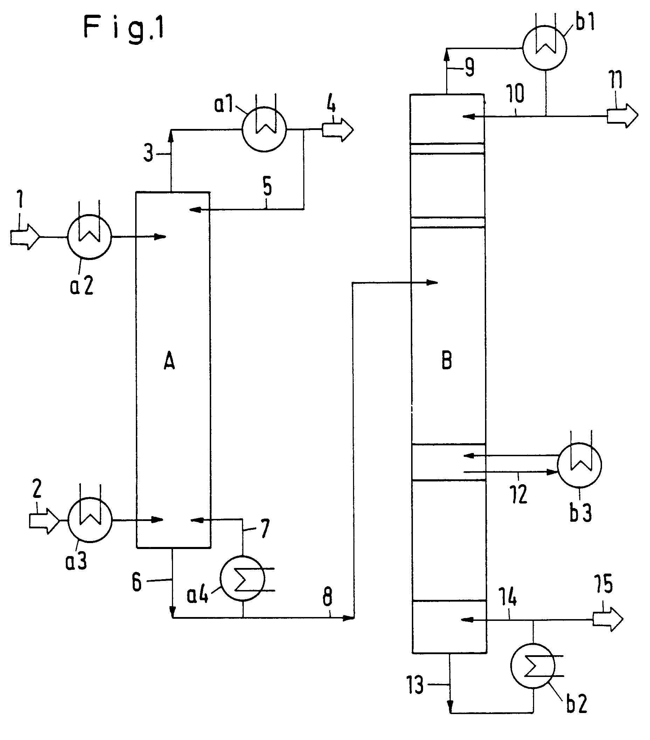

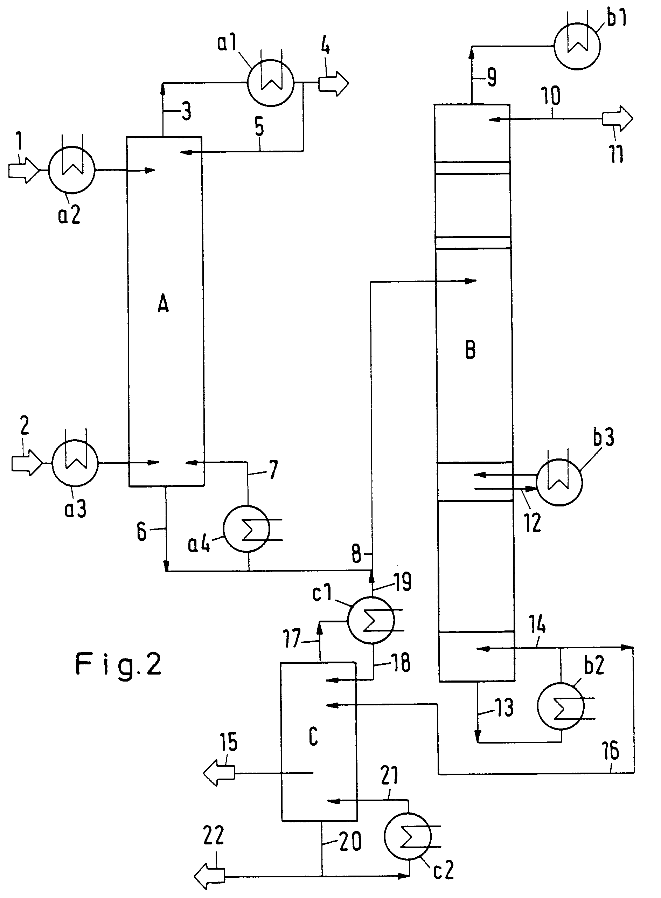

Es wird also hier ein schon bekanntes Verfahrensprinzip, die Durchführung von Umesterungsreaktionen in Kolonnen, auf ein spezielles Problem, nämlich auf die Umesterung von Alkylcarbonaten zu Arylcarbonaten angewendet. Besondere Maßnahmen technischen Handelns, die eine vorteilhaftere Durchführung der Umesterung unter Anpassung der Apparate und Vorgehensweisen an die oben angegebenen speziellen Probleme dieser schwierigen Umesterung erlauben, sind jedoch nicht angegeben. So wird beispielsweise die Art der Dosierung der beiden Edukte Alkylcarbonat und aromatische Hydroxyverbindung nicht klar definiert und auch keine vorteilhafte Fahrweise hervorgehoben. In einer Fahrweise nach Fig. 1 der EP 0 461 274 werden beispielsweise Gemische dieser beiden Edukte in den oberen Teil der Kolonne eingespeist, die leichtsiedenden Reaktionsprodukte, nämlich Alkohole und nichtumgesetztes Dialkylcarbonat, am Kopf der Kolonne und die schwersiedenden Reaktionsprodukte Alkylaryl- und Diarylcarbonate zusammen mit nichtumgesetztem Dialkylcarbonaten und aromatischen Hydroxyverbindungen am Fuß der Kolonne entnommen. In der Fahrweise nach Abb. 2 der EP 0 461 274 werden Gemische von Alkylcarbonaten und aromatischen Hydroxyverbindungen an zwei verscheidenen Punkten der Kolonne, nämlich im oberen und im unteren Drittel der Kolonne zugespeist und Edukt/Produkt-Gemische wie in Fahrweise nach Fig. 1 entnommen. Zwischen einer Führung der Edukte im Gleichstrom und im Gegenstrom wird weder im Text noch in den Beispielen klar unterschieden, obwohl sie von großem Einfluß auf das Verfahrensresultat sein können.

Ferner wird auf den Einfluß von Temperatur, Druck, Katalysatorkonzentration und Flüssigverweilzeit nicht eingegangen, sondern es werden nur sehr weite Bereichsangaben, auch in den bevorzugten Varianten gemacht, beispielsweise werden Temperaturbereiche von 100 bis 280°C, Druckbereiche von 0,1 bis 200 bar, Katalysatorkonzentrationen von 0,001 bis 50 Gew.-% und Flüssigverweilzeiten von 0,05 bis 2 Stunden angegeben.

Auf unterschiedliche und jeweils zu bevorzugende Verfahrensweisen für die einzelnen bei der Umsetzung von Dialkylcarbonaten zu Diarylcarbonaten auftretenden Reaktionen, beispielsweise die erste Umesterungsstufe von Dialkylcarbonaten mit aromatischen Hydroxyverbindungen zu Alkylarylcarbonaten nach Gleichung 1, die zweite Umesterungsstufe zu Diarylcaronbaten nach Gleichung 2 und die Disproportionierung gemäß Gleichung 3 wird in EP 0 461 274 nicht eingegangen.

$$\text{Alk - O - CO - O - Alk} + \text{Ar - OH} \rightarrow \text{Alk - O - CO - O - Ar} + \text{Alk - OH} \tag{1}$$

$$\text{Alk - O - CO - O - Ar} + \text{Ar - OH} \rightarrow \text{Ar - O - CO - O - Ar} + \text{Alk - OH} \tag{2}$$

$$2 \text{ Alk - O - CO - O - Ar} \rightarrow \text{Ar - O - CO - O - Ar} + \text{Alk - O - CO - O - Alk} \tag{3}$$

(Alk = Alkyl; Ar = Aryl)

Die Ausführungen dieser EP 0 461 274 führen den Fachmann zu dem Schluß, daß man die Umesterung von Phenolen mit Dialkylcarbonaten zwar in bekannter Weise kontinuierlich nach bekannten Verfahren in Kolonnen durchführen kann, daß es aber gleichgültig ist, nach welcher Variante, ob bei hoher oder niedriger Temperatur, im Gleich- oder Gegenstrom, bei niedrigem oder hohem Druck, mit großen oder kleinen Molverhältnissen usw. Kurz man muß daraus schließen, daß bei diesem besonderen Umesterungsproblem keine Möglichkeiten zur Verbesserung und vorteilhafteren Arbeitsweise bestehen. So können nur die angegebenen Beispiele zur Beurteilung des tatsächlichen Wertes dieser EP-Anmeldung herangezogen werden.

Aus diesen Beispielen ist erkennbar, daß bei der Umesterung von Dialkylcarbonaten mit Phenolen selbst bei relativ hohen Temperaturen, unter erhöhtem Druck und sogar bei molaren Dialkylcarbonatüberschüssen von mehr als 3 nur geringere Umsätze im Bereich von 10 bis 15 % (im besten Fall ca. 19 %) und vor allem nur sehr geringe Raumzeitausbeuten bis zu 0,02 kgl$^{-1}$h$^{-1}$ erzielt werden. Das ist verwunderlich, zumal sehr große Kolonnen darunter auch eine 20 bödige Kolonne mit 6 m Länge und ca. 300 l Volumen verwendet wurden. Der durch Dialkylcarbonatüberschüsse erzielbare höhere Phenolumsatz muß allerdings aus stöchiometrischen Gründen durch niedrigere Dialkylcarbonatumsätze erkauft werden. Das bedeutet, daß das am Kopf entnommene Dialkylcarbonat nur sehr geringe Mengen Alkohol enthält und daher in einem technischen Verfahren erheblich mehr unumgesetztes Ausgangsprodukt im Kreis gefahren und von den kleinen Mengen Reaktionsalkohol abgetrennt werden muß. Die geringen Raumzeitausbeuten würden bei vorgegebener Produktionsmenge pro Zeiteinheit sehr große Reaktoren und sehr große Destillationskapazitäten notwendig machen.

Die laut Beschreibung der EP 0 461 274 in einer nachgeschalteten zweiten Kolonne betriebene Disproportionierungsreaktion von Alkylarylcarbonaten gemäß Gleichung 3 läuft zwar mit höheren Ausbeuten ab, aber für eine technische Synthese von Diarylcarbonaten ist eine solche Disproportionierung von Alkylarylcarbonaten gegenüber der weiteren Umesterung mit Phenolen als wenig vorteilhaft anzusehen, da nur jedes zweite Alkylarylcarbonat-Molekül in das Diarylcarbonat-Endprodukt übergeführt und die andere Hälfte in das Ausgangsdialkylcarbonat zurückgeführt wird.

Aus den Beispielen 22 bis 30 der EP 0 461 274, in denen Umsetzungen in zwei hintereinander verschalteten Kolonnen beschrieben werden und die Zusammensetzung des Kopfprodukts aus der zweiten Kolonne als Feedstrom Nr. 6 in Abb. 4 oder 5 genannt ist, wird deutlich, daß trotz Anwesenheit von erheblichen Mengen an Phenolen kein Alkohol in der zweiten Reaktionsstufe gebildet wird und damit der Anteil der zweiten Umesterungsstufe gemäß Gleichung 2 nicht gegeben oder unbedeutend ist.

In einem technischen Prozeß zur Herstellung von Diarylcarbonaten, speziell von Diphenylcarbonat aus Dimethylcarbonat und Phenol, ist nicht nur der Phenolumsatz von Bedeutung, sondern auch die Dimethylcarbonatmenge, die zur Erzielung eines bestimmten Phenolumsatzes notwendig ist, und der sich daraus ergebende Dimethylcarbonatumsatz. In der Praxis wird man nur geringe Dimethylcarbonatumsätze und damit geringe Methanolkonzentrationen im Dimethylcarbonat am Kolonnenkopf erreichen können, beispielsweise solche von 5 bis 10 Gew.-% an Methanol. In der EP 0 461 274 geht man aber nun ohne Einschränkungen von reinem Dimethyl- oder Diethylcarbonat als Edukt aus. Dies ist angesichts der erhaltenen niedrigen Umsätze an Dialkylcarbonaten von nur wenigen Prozent verständlich und sicher unbedingt notwendig, weil aufgrund der ungünstigen Gleichgewichtslage beim Einsatz von alkoholhaltigen Dialkylcarbonaten die Umsätze noch niedriger und damit technisch untragbar würden. Das Methanol aber bildet mit Dimethylcarbonat ein nur mit großem Destillationsaufwand trennbares Azeotrop der Zusammensetzung 70 Gew.-% Methanol und 30 Gew.-% Dimethylcarbonat.

Einen besonders hohen Trennaufwand erfordert jedoch die Entfernung sehr geringer Mengen des Reaktionsmethanols aus dem Dimethylcarbonat-Produktstrom, wodurch die Rückführung des nicht umgesetzten Dimethylcarbonats in den Umesterungsprozeß in reiner Form nur unter großem Aufwand erreicht werden kann. Das ist auch von besonderer ökonomischer Bedeutung, da aufgrund nur geringer Dimethylcarbonätumsätze, die während eines Reaktordurchgangs erzielt werden können, die Dimethylcarbonat-Umlaufmengen sehr groß werden.

Ziel eines verbesserten Umesterungsverfahrens zur Herstellung von Diarylcarbonaten aus Dialkylcarbonaten und Phenolen müßte also sein, erstens deutliche Mengen Alkohole im Dialkylcarbonat-Eduktstrom tolerierbar zu machen und zweitens die Umesterungsstufe gemäß Gleichung 2, also die Umesterung von Phenol mit Alkylarylcarbonat zu Diarylcarbonat, zu begünstigen und die Disproportionierung von Alkylarylcarbonat zurückzudrängen.

Aus dem Massenwirkungsgesetz läßt sich ableiten, daß schon geringe Mengen an Alkoholen mit den schon gebildeten Arylcarbonaten aufgrund des sehr ungünstig liegenden Umesterungsgleichgewichts wieder in Richtung der Edukte abreagieren würden. Daher erscheint es aussichtslos, das oben angegebene 1. Ziel zu realisieren. Das haben wohl auch die Autoren der EP 0 461 274 angenommen.

Die Umesterung eines Alkylarylcarbonats mit Phenol zu Diarylcarbonat gemäß Gleichung 2 ist nach den Ergebnissen der EP 0 461 274 offensichtlich gegenüber der Disproportionierung zweier Alkylarylcarbonatmoleküle gemäß Gleichung 3 benachteiligt oder sogar völlig unterdrückt. Damit schien es auch höchst fraglich, ob das 2. Ziel erreicht werden kann. Für eine technische Synthese ist weiter eine Erhöhung der Raum-Zeit-Ausbeuten über die in der EP 0 461 274 genannten als ein 3. Ziel anzustreben, um die Größe der Apparate zu reduzieren. Auch dafür bietet EP 0 461 274 keine Lösung.

Überraschenderweise wurde nun gefunden, daß die Umsetzung von Phenolen mit Dialkylcarbonaten zu Diarylcarbonaten mit den angestrebten Verbesserungen in einem zweistufigen Prozeß erreichbar ist. Dabei setzt man zunächst Dialkylcarbonate, die gegebenenfalls auch deutliche Mengen des korrespondierenden Alkohols enthalten können, im Sinne einer "Gegenstromumesterung" (Reaktor A) mit Phenolen und das entstehende Produktgemisch in einem zweiten kolonnenartigen Reaktor im Sinne einer "Reaktionsdestillation" (Reaktor B) um (Fig. 1-2).

Es wurde ein Verfahren zur Herstellung von Diarylcarbonaten der Formel

$$Ar^1 - O - CO - O - Ar^1 \tag{I},$$

in der

$Ar^1$ nichtsubstituiertes Phenyl oder Naphthyl oder 1 bis 3fach substituiertes Phenyl, wobei die Substituenten gleich oder verschieden sind und zur Gruppe von $C_1$-$C_4$-Alkyl, $C_1$-$C_4$-Alkoxy und Halogen gehören, bedeutet,

durch Umesterung von 1 Mol von aromatischen Hydroxyverbindungen der Formel

$$Ar^1 - OH \tag{II},$$

worin $Ar^1$ die genannte Bedeutung hat,
mit 0,1 bis 10 Molen, bevorzugt mit 0,2 bis 5 Molen, besonders bevorzugt mit 0,5 bis 3 Molen Dialkylcarbonaten der Formel

$$R^1 - O - CO - O - R^1 \tag{III},$$

in der

$R^1$ geradkettiges oder verzweigtes $C_1$-$C_6$-Alkyl, Cyclopentyl oder Cyclohexyl bedeutet,

in Gegenwart von an sich bekannten Umesterungskatalysatoren in für Umesterungen bekannten Kolonnenapparaten gefunden, das dadurch gekennzeichnet ist, daß die Umesterungsreaktion in zwei hintereinander geschalteten Kolonnen in der Art durchgeführt wird, daß

a) in der ersten Kolonne die aromatische Hydroxyverbindung flüssig vom Kopf zum Sumpf geführt wird und das Dialkylcarbonat gasförmig diesem flüssigen Strom entgegengeführt wird, wobei die Temperatur im Bereich von 100 bis 300°C, bevorzugt 120 bis 250°C, besonders bevorzugt 150 bis 240°C und der Druck im Bereich von 0,5 bis 20 bar, bevorzugt 0,8 bis 15 bar, besonders bevorzugt 0,9 bis 10 bar, gehalten werden und das Dialkylcarbonat einen Anteil von 0 bis 5 Gew.-%, bevorzugt 0,1 bis 3 Gew.-%, besonders bevorzugt 0,2 bis 2 Gew.-%, des zugrundeliegenden Alkohols, bezogen auf das Gesamtgewicht von Dialkylcarbonat und Alkohol, enthält, daß

b) am Kopf der ersten Kolonne der dem Dialkylcarbonat zugrundeliegende und bei der Umesterung freiwerdende Alkohol gasförmig und am Sumpf der ersten Kolonne ein teilweise umgesetztes und im wesentlichen aus Alkylarylcarbonat bestehendes Umesterungsprodukt flüssig entnommen werden, daß

c) der Sumpfstrom der ersten Kolonne in den mittleren Teil einer zweiten Kolonne eingespeist wird, deren Temperatur im Bereich von 60 bis 320°C, bevorzugt 65 bis 305°C, besonders bevorzugt 65 bis 250°C und deren Druck im Bereich von 0,05 bis 10 bar, bevorzugt 0,1 bis 5 bar und besonders bevorzugt 0,2 bis 2 bar gehalten werden, wobei an diese zweite Kolonne in Abhängigkeit vom Siedepunkt des eingespeisten Sumpfstroms aus der ersten Kolonne ein solcher Temperatur-Gradient gelegt wird, daß das im eingespeisten Sumpfstrom enthaltene Alkylarylcarbonat aus dem mittleren Teil der zweiten Kolonne im wesentlichen weder zum Kopf noch zum Sumpf entweichen kann und daß

d) am Kopf der zweiten Kolonne ein Gemisch aus Dialkylcarbonat, dem zugrundeliegenden Alkohol und überschüssiger aromatischer Hydroxyverbindung und am Sumpf der zweiten Kolonne Diarylcarbonat, das Alkylarylcarbonat und aromatische Hydroxyverbindung enthalten kann, entnommen werden.

Bei der Bildung des Diarylcarbonats erfolgt die Umesterung von den aliphatischen zu den aromatischen Estergruppen in zwei Stufen, obwohl auch während der ersten Umesterungsstufe bereits etwas Diarylcarbonat gebildet werden kann. Die obige Gleichung 3 zeigt ferner eine Disproportionierungsreaktion, in welcher aus dem Alkylarylcarbonat sowohl das Dialkylcarbonat als auch das gewünschte Diarylcarbonat entstehen. Es ist ferner möglich, das Alkylarylcarbonat als das gewünschte Reaktionsprodukt zu erhalten, also nur die erste Umesterungsstufe in der ersten Kolonne zu betreiben. Dialkylcarbonate für das erfindungsgemäße Verfahren sind dem Fachmann bekannt und können nach bekannten Verfahren hergestellt werden. Geradkettiges oder verzweigtes $C_1$-$C_6$-Alkyl ist beispielsweise Methyl, Ethyl, Propyl, Isopropyl, Butyl, Isobutyl, Pentyl oder Hexyl.

Geradkettiges oder verzweigtes $C_1$-$C_4$-Alkoxy ist beispielsweise Methoxy, Ethoxy, Propoxy, Isopropoxy, Butoxy oder Isobutoxy.

Halogen ist beispielsweise Fluor, Chlor oder Brom, bevorzugt Fluor oder Chlor, besonders bevorzugt Chlor.

Die aromatische Estergruppe kann von einem Phenol oder einem Naphthol, bevorzugt von einem Phenol abgeleitet sein und in der angegebenen Weise ein- bis dreifach, bevorzugt ein- oder zweifach, besonders bevorzugt einfach substituiert und ganz besonders bevorzugt unsubstituiert sein.

Erfindungsgemäß einsetzbare aromatische Hydroxyverbindungen der Formel (II) sind beispielsweise nicht substituiertes Phenol, o-, m- oder p-Kresol, o-, m- oder p-Chlorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenol, 1-Naphthol und 2-Naphthol.

Bevorzugt einsetzbare aromatische Hydroxyverbindungen sind demnach allgemein solche der Formel

$$Ar^{11}\text{-OH} \qquad\qquad (IV),$$

in der

$Ar^{11}$      Phenyl oder einfach durch $C_1$-$C_4$-Alkyl, $C_1$-$C_4$-Alkoxy oder Chlor substituiertes Phenyl bedeutet.

Hierunter ist das nicht substituierte Phenol besonders bevorzugt.

Erfindungsgemäß einsetzbare Dialkylcarbonate der Formel (III) sind beispielsweise Dimethylcarbonat, Diethylcarbonat, Dipropylcarbonat, Dibutylcarbonat und Dihexylcarbonat. Bevorzugt einsetzbare Dialkylcarbonate sind Dimethyl- und Diethylcarbonat, besonders bevorzugt Dimethylcarbonat.

Erfindungsgemäß herstellbare Diarylcarbonate der Formel (I) sind beispielsweise Diphenylcarbonat, die isomeren Biskresylcarbonate, die isomeren Bis(chlorphenyl)-carbonate, die isomeren Bis(methoxyphenyl)-carbonate, die isomeren Bis(ethoxyphenyl)-carbonate, Bis(2,6-dimethylphenyl)-carbonat, Bis(2,4-dimethylphenyl)-carbonat, Di-1-naphthyl-carbonat und Di-2-naphthyl-carbonat.

Besonders bevorzugt herstellbares Diarylcarbonat ist Diphenylcarbonat.

Erfindungsgemäß als teilweise umgesetztes Umesterungsprodukt auftretende Aralkylarylcarbonate sind beispielsweise $C_1$-$C_6$-Alkyl-phenyl-carbonate, wie Methyl-phenyl-carbonat, Ethyl-phenyl-carbonat, Propyl-phenyl-carbonat, Butylphenyl-carbonat und Hexyl-phenyl-carbonat, $C_1$-$C_6$-Alkyl(o-, m-, p-kresyl)-carbonate, wie Methyl-(o-kresyl)-carbonat, Methy-(p-kresyl)-carbonat, Ethyl-(o-kresyl)-carbonat, Ethyl-(p-kresyl)-carbonat, $C_1$-$C_6$-Alkyl-(o-, m-p-Chlorphenyl)-carbonate, wie Methyl- oder Ethyl-(p-chlorphenyl)-carbonat und analoge Verbindungen. Besonders bevorzugte Alkylarylcarbonate sind Methyl-phenyl-carbonat und Ethyl-phenyl-carbonat.

Das erfindungsgemäße Verfahren wird mit Hilfe der beigefügten Fig. 1 und Fig. 2 wie folgt erläutert:

In einem kolonnenartigen Reaktor (A) werden die beiden Edukte, nämlich ein Phenol und ein Dialkylcarbonat, das gegebenenfalls geringe Mengen des korrespondierenden Reaktionsalkohols enthält, auf der überwiegenden Reaktorstrecke im Sinne einer "Gegenstromumesterung" gegeneinander geführt und zu Alkylarylcarbonaten und geringen Anteilen Diarylcarbonaten umgesetzt. Die wesentlichen Merkmale der im Sinne der Erfindung als "Gegenstromumesterung" bezeichneten Fahrweise sind dabei die folgenden: Die Edukte und die entstehenden Produkte müssen jeweils paarweise im Gegenstrom zueinander geführt werden, d.h. das eine Edukt, hier das Phenol, wird am oberen Ende der Kolonne (1) in flüssiger Form und das Dialkylcarbonat-Alkoholgemisch am unteren Ende der Kolonne (2) gasförmig eingespeist, der leichtflüchtige Reaktionsalkohol wird zusammen mit noch nicht umgesetztem Dialkylcarbonat am Kopf der Kolonne (4) und das gebildete schwererflüchtige Alkylarylcarbonat zusammen mit noch nicht umgesetztem Phenol am Fuß der Kolonne (8) entnommen. Es müssen eine Flüssigphase, die im vorliegenden Fall im wesentlichen aus dem Phenol und dem gebildeten Alkylarylcarbonat besteht, und eine Gasphase, die im wesentlichen das leichtflüchtige Dialkylcarbonat und den Reaktionsalkohol enthält, nebeneinander vorliegen, wobei die Reaktion in der Flüssigphase durch Lösen des gasförmig dosierten Dialkylcarbonats im flüssigen Phenol eintritt. Die Temperaturen und Drücke müssen also so aufeinander abgestimmt sein, daß keine wesentliche Verdampfung der Flüssigphase eintritt.

In einen zweiten Reaktor B wird das im Reaktor A erzeugte Alkylarylcarbonat im Sinne einer "Reaktionsdestillation" weiter umgesetzt. Die für eine "Reaktionsdestillation" im Sinne der Erfindung wesentlichen Merkmale sind die folgenden: Das Alkylarylcarbonat-Zwischenprodukt wird durch einen speziell gewählten Temperaturgradienten im Reaktor B weitgehend daran gehindert, den Reaktionsteil des Reaktors nach oben oder nach unten zu verlassen. Die leichtflüchtigen Reaktionsprodukte, hier Reaktionsalkohol und Dialkylcarbonat, werden am Kopf der Kolonne B über (11), das schwerflüchtige Reaktionsprodukt, hier das Diarylcarbonat, wird am Fuß der Kolonne über (15) entnommen. Das überschüssige Phenol kann entweder zusammen mit den Diarylcarbonat-Endprodukten am Fuß des Reaktors B bei (15) oder bevorzugt zusammen mit den Leichtsiederprodukten am Kopf der Kolonne B über (11) entnommen werden. Die Bedingungen werden dabei bevorzugt so gewählt, daß in der Reaktionszone des Reaktors B ein hoher Phenolüberschuß von 2 bis 20, bevorzugt von 3 bis 15, besonders bevorzugt von 3 bis 10 Molen pro Mol Alkylarylcarbonat erhalten bleibt.

In Fig. 1 und Fig. 2 bedeuten weiterhin (3) und (9) die Entnahmeleitungen für gasförmiges Kopfprodukt aus A bzw. B, das jeweils in Kondensatoren (a1) bzw. (b1) verflüssigt wird und teilweise über (5) bzw. (10) als Rückfluß nach A bzw. B zurückgeführt wird. (a2) und (a3) sind Vorwärmer, (a4) und (b2) sind Sumpfumlauf-Erhitzer; über (6) bzw. (13) werden die Sumpfabläufe von A bzw. B entnommen, zum Teil über (7) bzw. (14) nach A bzw. B zurückgeführt und zum anderen Teil als teilweise umgesetztes Umesterungsprodukt von A über (8) nach B geführt bzw. über (15) als rohes Diarylcarbonat entnommen.

Die besondere Fahrweise gemäß Fig. 2 wird weiter unten erläutert; soweit gleiche Bezugszeichen in Fig. 1 und Fig. 2 verwendet werden, haben sie die gleiche Bedeutung.

Mit der oben beschriebenen, auf das spezielle Umesterungsproblem angepaßten Verfahrensweise in zwei hintereinander geschalteten kolonnenartigen Reaktoren, in denen verschiedene, auf die jeweilige Reaktionsstufe angepaßte Fahrweisen, realisiert werden, erreicht man im Vergleich zum Stand der Technik erhöhte Dialkylcarbonat- und Phenolumsätze und größere Raum-Zeit-Ausbeuten. Insbesondere sind beim erfindungsgemäßen Verfahren auch deutliche Mengen des korrespondierenden Alkohols im Dialkylcarbonat-Eduktstrom tolerierbar, so daß nicht nur reine Dialkylcarbonate in das Verfahren einsetzbar sind, sondern auch Gemische mit geringen Mengen des Reaktionsalkohols, beispielsweise aus einer Dimethylcarbonat/Methanol-Trenndestillation. Im zweiten Reaktor reagiert das Alkylarylcarbonat-Zwischenprodukt nicht nur im Sinne einer Disproportionierung gemäß Gleichung 3 mit sich selbst zu Diarylcarbonaten und Dialkylcarbonaten, sondern zusätzlich in deutlichem Ausmaß mit einem Phenol im Sinne einer Umesterungsreaktion gemäß Gleichung 2 zu Diarylcarbonaten und Alkoholen, wodurch deutlich weniger Dialkylcarbonat als Leichtersiederprodukt anfällt.

Das am Kopf der Kolonne B entnommene Dialkylcarbonat/Alkohol-Gemisch besteht aus 25 bis 98 Gew.-%, bevorzugt 50 bis 95 Gew.-%, besonders bevorzugt 75 bis 92 Gew.-%, Dialkylcarbonat der Formel (III); der Rest zu 100 Gew.-% besteht aus dem zugrundeliegenden Alkohol

$$R^1\text{-OH} \qquad\qquad (V),$$

worin $R^1$ die obige Bedeutung hat.

Dieses Dialkylcarbonat/Alkohol-Gemisch steht seinerseits im Gemisch mit überschüssiger aromatischer Hydroxyverbindung, deren Anteil von dem aus der Kolonne A eindosierten Überschuß an dieser aromatischen Hydroxyverbindung und dem an die Kolonne angelegten Temperaturprofil abhängt.

Das am Sumpf der Kolonne B entnommene rohe Diarylcarbonat hat eine Reinheit von 50 bis 100 Gew.-%, bevorzugt 80 bis 99 Gew.-%, besonders bevorzugt 85 bis 98 Gew.-%; der Rest zu 100 Gew.-% besteht aus Alkylarylcarbonat der Formel

$$Ar^1\text{-O-CO-O-}R^1 \qquad\qquad\text{(VI)},$$

in der $Ar^1$ und $R^1$ die obige Bedeutung haben.

Überschüssig eingesetztes und in der Kolonne A nicht völlig umgesetztes Phenol (II) soll möglichst im mittleren Teil von B gehalten werden, um mit (VI) im Sinne der obigen Gleichung 2 zu reagieren. Soll Phenol aus B abgezogen werden, ist dies in beschriebener Weise über Kopf oder über den Sumpf möglich. Ein solches abzuziehendes Phenol ergänzt dann die oben beschriebenen Kopf- bzw. Sumpfabläufe von B.

Die in der ersten Reaktionsstufe des oben beschriebenen 2-stufigen Verfahrens einzusetzende und als "Gegenstromkolonne" bezeichnete Kolonne A stellt im einfachsten Fall ein isotherm beheiztes oder bevorzugt adiabatisch isoliertes, mit üblichen, für Destillationen zu verwendenden Füllkörpern oder Packungen gefülltes Rohr dar. Die Kolonne kann am unteren Ende ein bei höheren Temperaturen arbeitendes Abtriebsteil besitzen, in dem eine weitgehende bis vollständige Separierung des eindosierten Dialkylcarbonats von der herabrieselnden Flüssigphase erfolgt, wobei es wieder dampfförmig in den Umesterungsbereich der Kolonne geführt wird.

Weiterhin kann die Kolonne A am oberen Teil ein Verstärkerteil besitzen, das mitverdampftes Phenol oder Alkylphenylcarbonat von den leichtsiedenden Reaktionsalkoholen, bzw. Dialkylcarbonaten abtrennt und flüssig in den Umesterungsteil der Kolonne zurückführt. Auf Verstärker- bzw. Abtriebsteil kann jedoch auch verzichtet werden.

Die zu verwendenden Füllkörper bzw. geordneten Packungen für die Kolonnen A und B sind die für Destillationen an sich üblichen, wie sie beispielsweise in Ullmann's Encyclopädie der Technischen Chemie, 4. Aufl., Bd. 2, S. 528 ff. oder in den Firmenschriften der in Frage kommenden Apparatebaufirmen beschrieben sind. Beispielsweise seien genannt: Raschig- oder Pallringe, Berl-, Intalox- oder Torussättel, Interpackkörper aus verschiedenen Materialien, wie Glas, Steinzeug, Porzellan, Edelstahl, Kunststofff, die insbesondere bei der Verwendung von Metall, gewebe- oder maschenartig verarbeitet sein können. Bevorzugt sind Füllkörper und geordnete Packungen, die eine große Oberfläche, eine gute Benetzung sowie ausreichende Verweilzeit der Flüssigphase aufweisen. Diese sind beispielsweise Pall- und Novolaxringe, Berlsättel, BX-Packungen, Montz-Pak, Mellapak, Melladur, Kerapak und CY-Packungen.

Für die Kolonnen A und B sind jedoch nicht nur Füllkörperkolonnen geeignet, sondern auch solche mit festen Einbauten. Geeignet sind allgemein Bodenkolonnen, z.B. solche mit Sieb-, Glocken-, Ventil-, Tunnel- und Zentritugalböden, die weiterum in unterschiedlichen Ausführungen vorliegen können. Hierunter sind solche mit Glocken- bzw. Ventilböden mit hohen Verweilzeiten bei gutem Stoffaustäusch, beispielsweise Glockenbodenkolonnen mit hohen Überlaufwehren, bevorzugt.

Die theoretische Bodenzahl der als Reaktor A zu verwendenden Kolonne beträgt 3 bis 50, bevorzugt 3 bis 30 und besonders bevorzugt 5 bis 20 Böden, der Flüssigkeits-Hold-up 1 bis 80 %, bevorzugt 10 bis 75 %, besonders bevorzugt 15 bis 70 % und ganz besonders bevorzugt 15-50 % des Kolonneninnenvolumens. Die genauere Auslegung des Umesterungs-, und des gegebenenfalls zu verwendendes Abtriebs- und des Verstarkerteils kann vom Fachmann vorgenommen werden.

Die Kolonne A wird so betrieben, daß man in die obere Hälfte, bevorzugt in das obere Drittel, einen Phenolstrom (1), der gegebenenfalls den Katalysator gelöst enthalten kann, bevorzugt mit der an dieser Stelle der Kolonne herrschenden Temperatur, flüssig eindosiert.

In die untere Hälfte der Kolonne A, bevorzugt oberhalb einer gegebenenfalls vorhandenen Abtriebszone, wird über (2) ein Dialkylcarbonatstrom, in der Regel in Dampfform, mit Temperaturen von 120 bis 220°C eindosiert Dieser Dialkylcarbonatstrom enthält 0 bis 5 Gew.-%, bevorzugt 0,1 bis 3 Gew.-% und besonders bevorzugt 0,2 bis 2 Gew.-%, des korrespondierenden Alkohols der Formel (IV). Die Energie zur Verdampfung und Überhitzung des Dialkylcarbonatstroms kann über einen separaten, außenliegenden oder in der Säule integrierten Wärmetauscher aufgebracht werden.

Nach Passieren der Umesterungszone wird der Reaktionsalkohol, gegebenenfalls nach Durchlaufen einer Verstärkerzone, am Kopf der Kolonne bei (4) entnommen. Er enthält im allgemeinen noch überschüssiges oder nicht umgesetztes Dialkylcarbonat und bei nicht vorhandenem Verstärkerteil auch gewisse Mengen der aromatischen Hydroxyverbindung.

Nach Passieren der Umesterungszone und eines gegebenenfalls vorhandenen Abtriebsteils tritt am Fuße der Kolonne A bei (6) ein Gemisch von Alkylarylcarbonat, mit überschüssigem oder nichtumgesetztem Phenol, mit gegebenenfalls geringen Mengen von schon gebildetem Diarylcarbonat, mit gegebenenfalls löslichen Katalysatoren und bei einer Fahrweise ohne Abtriebsteil auch mit Dialkylcarbonat aus. Das Sumpfprodukt wird in bevorzugter Weise direkt über (8) in die zweite Umesterungskolonne B eingespeist, kann aber auch vorher in geeigneter Weise aufkonzentriert werden, d.h., auf einen gewünschten Gehalt an Alkylarylcarbonat, Phenol und Dialkylcarbonat gebracht werden.

Im Rahmen des erfindungsgemäßen Umesterungsverfahren ist es zweckmäßig, die für die Reaktion in Reaktor A notwendige Energie nicht nur über innen- oder außenliegende Wärmetauscher irgendwelcher Art, sondern bevorzugt sowohl mit dem flüssigen Strom der aromatischen Hydroxyverbindung, als auch mit dem gasförmig eindosierten Dialkylcarbonat einzubringen. Weiterhin können in den Reaktor zum Ausgleich von Reaktionswärmen innen- oder außenlegende Wärmetauscher eingebaut werden. Die Kolonne weist über die gesamte Länge einen mehr oder weniger großen Temperaturgradienten auf.

Der Katalysator wird bevorzugt zusammen mit dem Phenol-Eduktstrom in gelöster oder suspendierter Form in die Kolonne A über (1) eingebracht. Alternativ kann der Katalysator auch separat beispielsweise im Reaktionsalkohol oder einem geeigneten systemfremden, inerten Lösungsmittel zudosiert werden. Im Falle der Verwendung heterogener Katalysatoren können diese im Gemisch mit den genannten Füllkörpern in geeigneter Form anstelle von Füllkörpern oder als Schüttung auf eingebaute Kolonnenböden eingesetzt werden.

Das Molverhältnis der eingesetzten Edukte in der Kolonne variiert von 0,1-10 Mol, bevorzugt von 0,2-5 Mol und besonders bevorzugt von 0,5-3 Mol Dialkylcarbonat pro Mol eingesetztem Phenol.

Die erste Umesterungsstufe im Reaktor A kann bei Temperaturen von 100-300°C, bevorzugt bei Temperaturen von 120-250°C und besonders bevorzugt bei Temperaturen von 150-240°C in der Kolonne durchgeführt werden. Ein in bevorzugter Weise anzulegender Temperaturgradient liegt im angegebenen Temperaturbereich und steigt vom Kolonnenkopf in Richtung Kolonnenfuß. Dabei muß gewährleistet sein, daß die Reaktionstemperatur im Umesterungsbereich nicht oberhalb der Verdampfungstemperatur des eingesetzten Phenols liegt. Es ist deshalb von Vorteil, die erfindungsgemäße Umesterung nicht nur bei Normaldruck, sondern auch bei erhöhtem oder erniedrigtem Druck von 500 mbar bis zu 20 bar durchzuführen. Ein bevorzugter Druckbereich liegt zwischen 0,8 und 15 bar, ein besonders bevorzugter Druckbereich liegt zwischen 0,9 und 10 bar.

Die Raum-Zeit-Belastung der Kolonne liegt bei 0,05-5 g Gesamtmenge der Reaktionsteilnehmer pro ml wirksames Kolonnenvolumen pro Stunde, bevorzugt bei 0,1-3 g/ml/h, besonders bevorzugt bei 0,2-2 g/ml/h; das wirksame Kolonnenvolumen ist hierbei das der Füllkörperpackung oder das Volumen, in welchem sich feste Einbauten befinden.

Der in der zweiten Reaktionsstufe einzusetzende und als "Reaktionskolonne" bezeichnete Reaktor B (Fig. 1-2) besteht aus einem kolonnenartigen Rohr dem ein Temperaturprofil angelegt wird, das von oben nach unten gesehen ansteigend einen Temperaturbereich von 50 bis 320°C, bevorzugt 65 bis 305°C und besonders bevorzugt von 65 bis 250°C, umfaßt Zur Einstellung der Temperaturgradienten in den einzelnen Abschnitten des kolonnenartigen Reaktors können diese Abschnitte mit einer Isolierung bzw. einer Thermostatisierung versehen werden. Die Thermostatisierung kann hierbei je nach Bedarf eine Heizung oder eine Kühlung darstellen. Der Reaktor B kann in verschiedenen Abschnitten seiner Gesamtlänge, entsprechend den Gas- und Flüssigbelastungen und den benötigten Verweilzeiten aufgeweitet oder verengt sein.

Für den mittleren Teil von B, den Reaktionsbereich, sind feste Einbauten bevorzugt, für die Teile, in denen Trennungen stattfinden, dagegen Füllkörper und feste Packungen.

Am unteren Ende der Kolonne B sind ein oder mehrere, gegebenenfalls durch adiabatisch isolierte Kolonnenteile getrennte Verdampfer angeordnet. Diese Verdampfer können innerhalb oder bevorzugt außerhalb der Kolonne angeordnet sein. In einer technischen Ausführung der Erfindung werden in der Technik übliche Apparate wie Umlaufverdampfer, Fallfilmverdampfer und Wendelrohrverdampfer verwendet.

Oberhalb der Verdampferzone, in dem als "Reaktionszone" bezeichneten mittleren Bereich, werden bevorzugt feste Einbauten und besonders bevorzugt solche mit großem Flüssigkeits-Hold Up benutzt, beispielsweise Glockenböden mit hohen Überlaufwehren, wie in DE 2 503 195 beschrieben. Die theroretische Bodenzahl in diesem Bereich beträgt 2 bis 50, bevorzugt 2 bis 25 und besonders bevorzugt 2 bis 15. Der Flüssigkeits-Hold Up in diesem Bereich beträgt 5 bis 80 %, bevorzugt 10 bis 75 %, besonders bevorzugt 15 bis 70 % und ganz besonders bevorzugt 15-50 % des Innenvolumens der Einbauten.

Wiederum oberhalb dieses Bereichs ist die Kolonne mit weiteren, im besonderen Maße für destillative Stofftrennungen geeigneten Füllkörpern oder Einbauten ausgestattet. Am oberen Ende der Kolonne B ist bevorzugt ein Verstärkerteil angeordnet, mit dem ein gezielter Rücklauf der Kolonne einstellbar ist.

Die Kolonne B wird (Fig. 1-2) so betrieben, daß man oberhalb der "Reaktionszone" über die Leitung (8) einen aus der Kolonne A bei (6) entnommen Strom aus Alkylarylcarbonat und aromatischer Hydroxyverbindung, der gegebenenfalls geringe Mengen Diarylcarbonat, Dialkylcarbonat und einen Umesterungskatalysator enthalten kann, flüssig eindosiert. Dieser Strom durchläuft die "Reaktionszone" und wird dort teilweise in Diarylcarbonat verwandelt, und die noch nicht umgesetzten Reden werden mit Hilfe der beschriebenen Verdampfer gasförmig zurück in die Reaktionszone und die oberen Teile der Kolonne B transportiert. Diese kondensieren dort und setzen sich erneut zum Diarylcarbonat-Endprodukt um. Das Diarylcarbonat-Endprodukt wird als höchst siedende Reaktionskomponente im Sumpfbereich der Kolonne angereichert und dort über (15) zusammen mit gegebenenfalls homogen gelöstem Katalysator und geringen Mengen Alkylphenylcarbonat und aromatischer Hydroxyverbindung ausgespeist.

Die leichtflüchtigen Reaktionsprodukte aus der Disproportionierungsreaktion gemäß Gleichung 3, nämlich Dialkylcarbonate der Formel III, bzw. aus der zweiten Umesterungsstufe gemäß Gleichung 2, nämlich die Alkohole der Formel (IV), werden am Kopf der Kolonne B über (11) entnommen. Die im Überschuß vorhandenen oder nicht umgesetzten Phenole der Formel (II) können entweder am Fuß der Kolonne über (15) zusammen mit dem Diarylcarbonat-Endprodukt der Formel (I) oder in einer bevorzugten Fahrweise zusammen mit den Leichtsiederprodukten am Kopf der Kolonne B über (11) ausgespeist werden.

In einer besonderen Fahrweise (Fig. 2) kann das im wesentlichen aus Diarylcarbonat bestehende Rohproduktgemisch (16) in einem nachgeschalteten separaten Reaktor C bei Drücken von 0,05 bis 1,0 bar noch weiter umgesetzt und aufgetrennt werden, wobei im Seitenstrom dieser Kolonne über (15) reines Diarylcarbonat, am Fuß der Kolonne ein Diarylcarbonat-haltiger Katalysatorsumpf (22) und über Kopf bei (19) die nicht umgesetzten Edukte bzw. gebildeten leichtsiedenden Produkte entnommen werden. Der Katalysatorsumpf (22) läßt sich an geeigneter Stelle in den Prozeß zurückführen, z.B. in den Reaktor A über (1) oder in den Reaktor B über (8). Bei teilweiser Desaktivierung ist es natürlich auch möglich, einen Teil des Katalysatorsumpfs zu entnehmen und den entfernten Teil an geeigneter Stelle z.B. über (1) durch frischen Katalysator zu ersetzen. Das als Kopfprodukt der Kolonne C anfallende Gemisch (19) läßt sich seitlich in den Produktstrom (8), gegebenenfalls nach Angleichung des Drucks, in den Umesterungsprozeß zurückführen.

Bei der Verwendung von homogen gelösten oder suspendierten Katalysatoren werden im wesentlichen die schon in der Kolonne A wirksamen, im Sumpfprodukt (6) enthaltenen Katalysatoren über (8) in die Kolonne B eingespeist und dadurch in der Reaktorzone von B wirksam. Darüber hinaus besteht aber auch die Möglichkeit, an einer Stelle oberhalb der Reaktionszone zusätzlichen Katalysator derselben Art oder einen zweiten Katalysator in die Kolonne B einzuspeisen. Beim Einsatz von heterogenen Katalysatoren können diese im Gemisch mit den genannten Füllkörpern, in geeigneter Form anstelle von Füllkörpern oder in bevorzugter Weise als Schüttung auf eingebaute Kolonnenböden eingesetzt werden.

Die Reaktion in der Kolonne B wird bei einem Druck zwischen 50 mbar und 10 bar, bevorzugt zwischen 0,1 und 5 bar und besonders bevorzugt zwischen 0,2 und 2 bar, durchgeführt. Die Temperaturen in dem als Reaktionszone bezeichneten Bereich $B_1$ der Kolonne B liegen zwischen 100 und 300°C, bevorzugt zwischen 120 und 280°C und besonders bevorzugt zwischen 150 und 260°C.

Als Katalysatoren kommen heterogene und homogene in Frage. Die heterogenen Katalysatoren können dabei als Festbett innerhalb der Kolonne angeordnet sein. Sie können aber auch in Form einer Suspension im oberen bis mittleren Teil der Kolonne, gegebenenfalls als Gemisch mit Teilen der Ausgangsstoffe, eingesetzt werden und gelangen gemeinsam mit den schwerflüchtigen Reaktionsprodukten am unteren Ende wieder aus der Kolonne; sie können nach Abtrennung erneut eingesetzt werden. Homogene Katalysatoren werden ebenfalls im oberen bis mittleren Teil der Kolonne eingesetzt und können hierbei als Gemisch mit der aromatischen Hydroxyverbindung eingesetzt werden.

Für alle erfindungsgemäßen Reaktionsschritte können die gleichen Katalysatoren eingesetzt werden. Diese sind aus der Literatur bekannte Umesterungskatalysatoren für die Dialkylcarbonat-Phenol-Umesterung, wie z.B. Hydride, Oxide, Hydroxide, Alkoholate, Amide und andere Salze von Alkali- und Erdalkalimetallen (US 3.642.858; US 3.803.201; EP 1082), wie von Lithium, Natrium, Kalium, Rubidium, Caesium, Magnesium und Calcium, bevorzugt Lithium, Natrium, Kalium, Magnesium und Calcium und besonders bevorzugt Lithium, Natrium und Kalium. Salze der Alkali- und Erdalkalimetalle können auch solche von organischen oder anorganischen Säuren sein, wie von Essigsäure, Propionsäure, Buttersäure, Benzoesäure, Stearinsäure, Kohlensäure (Carbonate oder Hydrogencarbonate), Phosphorsäure, Blausäure, Rhodanwasserstoffsäure, Borsäure, Zinnsäure, $C_1$-$C_4$-Stannonsäuren oder Antimonsäure. In bevorzugter Weise kommen als Verbindungen der Alkaliund Erdalkalimetalle die Oxide, Hydroxide, Alkoholate, Acetate, Propionate, Benzoate, Carbonate und Hydrogencarbonate in Frage, in besonders bevorzugter Weise werden Hydroxide, Alkoholate, Acetate, Benzoate oder Carbonate eingesetzt.

Die genannten Alkali- oder Erdalkalimetallverbindungen werden in Mengen von 0,001 bis 2 Gew.-%, bevorzugt 0,005 bis 0,9 Gew.-% und besonders bevorzugt 0,01 bis 0,5 Gew.-%, bezogen auf das umzusetzende Reaktionsgemisch, eingesetzt.

Weitere erfindungsgemäß einsetzbare Katalysatoren sind Lewis-saure Metallverbindungen wie $AlX_3$, $TiX_3$, $UX_4$, $TiX_4$, $VOX_3$, $VX_5$, $ZnX_2$, $FeX_3$ und $SnX_4$, worin X für Halogen, Acetoxy, Alkoxy oder Aryloxy steht (DE-OS 25 28 412, 2 552 907), beispielsweise Titantetrachlorid, Titantetraphenoxid, Titantetraethoxid, Titantetraisopropylat, Titantetradodecylat, Zinnterailsooctylat und Aluminiumtriisopropylat, weiterhin zinnorganische Verbindungen der allgemeinen Formel $(R^{11})_{4-x}$-$Sn(Y)_x$, in der Y für einen Rest $OCOR^{12}$, OH oder OR steht, wobei $R^{12}$ $C_1$-$C_{12}$-Alkyl, $C_6$-$C_{12}$-Aryl oder $C_7$-$C_{13}$-Alkylaryl bedeutet und $R^{11}$ unabhängig von $R^{12}$ die Bedeutung von $R^{12}$ hat und x eine ganze Zahl von 1 bis 3 bedeutet, Dialkylzinnverbindungen mit 1 bis 12 C-Atomen im Alkylrest oder Bis-(trialkylzinn)verbindungen, beispielsweise Trimethylzinnacetat, Triethylzinnbenzoat, Tributylzinnacetat, Triphenylzinnacetat, Dibutylzinndiacetat, Dibutylzinndilaurat, Dioctylzinndilaurat, Dibutylzinnadipinat, Diburyldimethoxyzinn, Dimethylzinnglykolat, Dibutyldiethoxyzinn, Triethylzinnhydroxid, Hexaethylstannoxan, Hexabutylstannoxan, Dibutylzinnoxid, Dioctylzinnoxid, Butylzinntriisooctylat, Octylzinntriisooctylat, Butylstannonsäure und Octylstannonsäure in Mengen von 0,001 bis 20 Gew.-% (EP 879, EP 880, EP 39 452, DE-OS 3 445 555, JP 79/63023), polymere Zinnverbindungen der Formel -[-R,$R^{11}$Sn-O-]-, beispielsweise Poly[oxy(dibutylstannylen)], Poly[oxy(dioctylstannylen)], Poly[oxy(butylphenylstannylen)] und Poly[oxy(diphenylstannylen)] (DE-OS 3 445 552), polymere Hydroxystannoxane der Formel -[-RSn(OH)-O-]-, beispielsweise Poly(ethylhydroxystannoxan), Poly(butylhydroxystannoxan), Poly(octylhydroxystannoxan), Poly(undecylhydroxystannoxan) und Poly(dodecylhydroxystannoxane) in Mengen von 0,001 bis 20 Gew.-%, bevorzugt von 0,005 bis 5 Gew.-%, bezogen auf Kohlensäurediester (DE-OS 4 006 520).

Weitere erfindungsgemäß einsetzbare Zinnverbindungen sind Sn(II)oxide und besitzen die Formel

$$X\text{-}R_2Sn\text{-}O\text{-}R_2SnY,$$

worin

X und Y unabhängig voneinander OH, SCN, $OR^{11}$, $OCOR^{11}$ oder Halogen und R Alkyl, Aryl bedeuten soll (EP 0 338 760).

Als weitere erfindungsgemäß einsetzbare Katalysatoren kommen Bleiverbindungen, gegebenenfalls zusammen mit Triorganophosphanen, einer Chelatverbindung oder einem Alkalimetallhalogenid, beispielsweise $Pb(OH)_2 \cdot 2PbCO_3$, $Pb(OCO\text{-}CH_3)_2$, $Pb(OCO\text{-}CH_3)_2 \cdot 2LiCl$, $Pb(OCO\text{-}CH_3)_2 \cdot 2PPh_3$ in Mengen von 0,001 bis 1, bevorzugt von 0,005 bis 0,25 Mol pro Mol Carbonat (JP 57/176932, JP 01/093580), andere Blei(II)- und Blei(IV)-verbindungen, wie PbO, $PbO_2$, Mennige, Plumbite, und Plumbate (JP 01/093560), Eisen(III)acetat (JP 61/172852), weiterhin Kupfersalze und/oder Metallkomplexe, beispielsweise von Alkali, Zink, Titan und Eisen (JP 89/005588), Kombinationen aus Lewis-Säuren und Protonensäuren (DE-OS 3 445 553) oder Elementverbindungen von Sc, Cr, Mo, W, Mn, Au, Ga, In, Bi, Te und Lanthaniden (EP 338 760) in Frage.

Weiterhin sind in den erfindungsgemäßen Verfahren heterogene Katalysatorsysteme einsetzbar. Solche sind beispielsweise Mischoxide aus Silicium und Titan, die durch gemeinsame Hydrolyse von Silicium und Titanhalogeniden herstellbar sind (JP 54/125617) und Titandioxide mit hoher BET-Oberfläche >20 $m^2/g$ (DE-OS 4 036 594)).

Bevorzugt im erfindungsgemäßen Verfahren einsetzbare Katalystoren sind Zinn-, Titan- und Zirkoniumverbindungen und die oben genannten Alkali- und Erdalkaliverbindungen, besonders bevorzugt einsetzbare Katalysatoren sind Organozinnverbindungen und Titantetraalkyl- und -arylester.

Die einzusetzenden Katalysatormengen können sich teilweise von den in der Literatur genannten Mengen unterscheiden.

<u>Beispiele 1 - 6</u>

(Fig. 1, Reaktor A, Gegenstromumesterung)

In eine mit V4A-Maschendrahtringen (3x3 mm) gefüllte isotherm auf 175°C thermostatisierte Kolonne von 185 cm Länge und 28 mm Durchmesser dosierte man am Kopf kontinuierlich ein auf 160°C vorgeheiztes flüssiges Gemisch aus Phenol und dem jeweils verwendeten Katalysator. Diesem Flüssigkeitsstrom schickte man gasförmiges Dimethylcarbonat oder gasförmige Gemische aus Dimethylcarbonat und Methanol entgegen, das/die in einem separaten Apparat verdampft und 35 cm oberhalb des Kolonnenfußes in die Kolonne eingespeist wurde(n). Am oberen Ende der Kolonne, die ein kurzes Verstarkerteil besaß (15 cm adiabatische Kolonne mit Rücklaufteiler) nahm man kontinuierlich ein Gemisch aus Methanol und Dimethylcarbonat (Kopfprodukt), am Fuß der Kolonne ein Gemisch aus Methylphenylcarbonat, Diphenylcarbonat, wenig Dimethylcarbonat und dem Katalysator (Sumpfprodukt) ab. Da kein separates Abtriebsteil vorhanden war, konnte das Dimethylcarbonat aus dem Sumpfprodukt nicht vollständig entfernt werden, was zu einer Verdünnung der Produktkonzentration im Sumpf führte. Die Selektivitäten bezüglich der Methylphenyl- und der Diphenylcarbonatbildung betrug für alle Beispiele >99 %.

In der folgenden Tabelle 1 sind Einsatzmengen, Reaktionsbedingungen und Versuchsergebnisse zusammengestellt. Die Ergebnisse wurden nach Einstellen konstanter Bedingungen durch Mitteln über mehrere Messungen bestimmt.

Tabelle 1:

| Beispiel Nr. | Edukte | | | Menge [g/h] | Sumpfprodukt Zusammensetzung [Gew.-%] | | | | Raum-Zeit-Ausbeute *) [g/ml*h] | Kopfprodukt | | |
|---|---|---|---|---|---|---|---|---|---|---|---|---|
| | Phenol [g/h] | Katalysator ([g/h]) | DMC/Meth DMC-Geh.[%] (Menge[g/h]) | | MPC | DPC | Phenol | DMC | | Menge [g/h] | Methanol [Gew.-%] | DMC [Gew.-%] |
| 1 | 150 | Butylstannonsäure (1,7) | 100 (150) | 180 | 11,7 | 1,1 | 75,0 | 12,2 | 0,022 | 120 | 4,3 | 95,7 |
| 2 | 150 | Octylstannonsäure (2,1) | 100 (150) | 175 | 13,9 | 1,5 | 76,5 | 8,2 | 0,025 | 125 | 4,8 | 95,2 |
| 3 | 250 | Octylstannonsäure (3,5) | 100 (250) | 295 | 10,3 | 1,1 | 78,3 | 10,2 | 0,032 | 209 | 3,5 | 96,5 |
| 4 | 250 | Octylstannonsäure (3,5) | 100 (500) | 292 | 10,8 | 1,1 | 78,8 | 9,4 | 0,033 | 460 | 1,7 | 98,3 |
| 5 | 150 | Octylstannonsäure (2,1) | 98 (150) | 175 | 10,1 | 1,1 | 80,6 | 8,3 | 0,020 | 125 | 5,8 | 94,2 |
| 6 | 150 | Octylstannonsäure (2,1) | 99 (150) | 180 | 11,4 | 1,2 | 78,9 | 8,4 | 0,024 | 120 | 5,4 | 94,6 |

*) Raumzeitausbeute für die MPC⁻ und DPC⁻Bildung als MPC gerechnet.
DMC = Dimethylcarbonat
MPC = Methylphenylcarbonat
DPC = Diphenylcarbonat

Beispiel 7

(Fig. 1, Reaktor A, Gegenstromumesterung)

In eine 350 cm lange Füllkörperkolonne mit 28 mm Innendurchmesser, die sonst, wie in den Beispielen 1-6 beschrieben, ausgestattet war, dosierte man unter den dort beschriebenen Bedingungen kontinuierlich am Kopf ein Gemisch aus 100 g/h Phenol und 1,4 g/h Octylstannonsäure und im Gegenstrom 100 g/h dampfförmiges Dimethylcarbonat. Am oberen Ende der Kolonne entnahm man kontinuierlich 75 g/h eines Gemisches aus 12,3 Gew.-% Methanol und 87,7 Gew.-% Dimethylcarbonat und am Fuß der Kolonne 125 g/h eines Gemisches aus 26,8 Gew.-% Methylphenylcarbonat, 5,4 Gew.-% Diphenylcarbonat, 60,8 Gew.-% Phenol, 7,0 Gew.-% Dimethylcarbonat und dem Katalysator. Das entsprach einem Phenolumsatz von 26 %. Die Selektivität bezüglich der Methylphenyl- und Diphenylcarbonatbildung betrug >99%.

Beispiel 8

(Fig. 1, Reaktor A, Gegenstromumesterung)

Wie in den Beispielen 1-6 beschrieben, setzte man ein Gemisch aus 150 g/h Phenol und 2,1 g/h Octylstannonsäure im Gegenstrom mit 150 g/h dampfförmigem Diethylcarbonat um. Am oberen Ende der Kolonne entnahm man nach Einstellen konstanter Bedingungen kontinuierlich 115 g/h eines Gemisches aus 6,5 Gew.-% Ethanol und 93,5 Gew.-% Diethylcarbonat und am Fuß der Kolonne 185 g/h eines Gemisches aus 12,4 Gew.-% Ethylphenylcarbonat, 0,6 Gew.-% Diphenylcarbonat, 75,0 Gew.-% Phenol, 12,0 Gew.-% Diethylcarbonat und dem Katalysator. Das entsprach einem Phenolumsatz von 9,1 %. Die Selektivität bezüglich der Ethylphenyl- und Diphenylcarbonatumsatz betrug >99%.

Beispiele 9-14

(Fig. 1, Reaktor A, Gegenstromumesterung)

Es wurde im Unterschied zu der in den Beispielen 1-8 beschriebenen Füllkörperkolonne eine isotherm auf 175°C thermostatisierte Glasglockenbodenkolonne mit einer Länge von 72 cm und einem Durchmesser von 4,75 cm mit insgesamt 10 Böden und einem Flüssig-hold up von 240 ml verwendet. Am Fuß der Kolonne war ein 35 cm langes Eindosierstück und am Kopf der Kolonne ein 40 cm langes Verstarkerteil angeordnet. Die Reaktionen wurden wie in den Beispielen 1-6 durchgeführt. Die Selektivitäten bezüglich Methylphenyl- und Diphenylcarbonatbildung betrug für alle Beispiel >99 %.

In der Tabelle 2 sind Einsatzmengen, Reaktionsbedingungen und Versuchsergebnisse zusammengestellt. Die Ergebnisse wurden nach Einstellen konstanter Bedingungen durch Mitteln über mehrere Messungen ermittelt.

Tabelle 2:

| Beispiel Nr. | Edukte | | | Menge [g/h] | Sumpfprodukt Zusammensetzung [Gew.-%] | | | | Raum-Zeit-Ausbeute *) [g/ml*h] | Kopfprodukt | | |
|---|---|---|---|---|---|---|---|---|---|---|---|---|
| | Phenol [g/h] | Katalysator ([g/h]) | DMC/Meth DMC-Geh.[%] (Menge[g/h]) | | MPC | DPC | Phenol | DMC | | Menge [g/h] | Methanol [Gew.-%] | DMC [Gew.-%] |
| 9 | 250 | Octylstannonsäure (3,5) | 100 (250) | 280 | 12,5 | 5,1 | 79,7 | 2,6 | 0,055 | 220 | 5,3 | 94,7 |
| 10 | 150 | Octylstannonsäure (2,0) | 100 (150) | 170 | 12,2 | 4,4 | 78,5 | 4,9 | 0,031 | 130 | 5,1 | 94,9 |
| 11 | 250 | Dibutylzinnoxid (3,2) | 100 (250) | 275 | 10,7 | 3,0 | 81,4 | 4,8 | 0,041 | 225 | 3,8 | 96,2 |
| 12 | 250 | Titan(IV)isopropylat (3,7) | 100 (250) | 278 | 10,8 | 3,3 | 80,8 | 4,8 | 0,043 | 225 | 4,0 | 96,0 |
| 13 | 250 | Octylstannonsäure (3,5) | 98 (250) | 273 | 9,2 | 2,5 | 83,7 | 4,6 | 0,035 | 225 | 3,3 | 96,7 |
| 14 | 250 | Octylstannonsäure (3,5) | 99 (250) | 275 | 10,5 | 2,9 | 81,7 | 4,9 | 0,040 | 225 | 3,6 | 96,4 |

*) Raumzeitausbeute für die MPC⁻ und DPC⁻Bildung als MPC gerechnet.
DMC = Dimethylcarbonat
MPC = Methylphenylcarbonat
DPC = Diphenylcarbonat

Beispiel 15

(Fig. 1, Reaktor B, Reaktionsdestillation)

Es wurde ein kolonnenartiger Reaktor verwendet, der, von oben nach unten gesehen, folgendermaßen aufgebaut war:

- auf 95°C beheizter Dephlegmator von 20 cm Länge mit aufgesetztem einfachen Destillationskopf und Leichtsiederentnahmestelle;
- Edukteindosierstelle;
- isotherm auf 185°C thermostatisierte 10bödige Siebbodenkolonne von 65 cm Länge und 4,5 cm Innendurchmesser;
- isotherm auf 270°C thermostatisiertes, mit V4A-Maschendrahtringen (3x3 mm) gefülltes Rohr (Verdampfer 2) von 25 cm Länge und 2,8 cm Innendurchmesser;
- adiabatisch isolierte, mit V4A-Maschendrahtringen gefüllte Kolonne von 25 cm Länge und 2,8 cm Innendurchrmesser;
- isotherm auf 265°C thermostatisierter Wendelrohrverdampfer (Verdampfer 1) von 25 cm Länge mit Sumpfproduktentnahmestelle.

In diesen Reaktor wurde an der Eindosierstelle oberhalb der Siebbodenkolonne kontinuierlich ein Gemisch aus 75 g/h Methylphenylcarbonat, 175 g/h Phenol und 3 g/h Octylstannonsäure eindosiert. Gleichzeitig wurde diesem Eduktstrom ein schwacher Stickstoffstrom von $2[l \cdot h^{-1}]$ vom Wendelrohrverdampfer entgegengeführt. Nach dem Einstellen konstanter Bedingungen entnahm man am Kopf der Apparatur kontinuierlich 195 g/h eines Gemisches aus 2,2 Gew.-% Methanol, 13,7 Gew.-% Dimethylcarbonat und 84,1 Gew.-% Phenol und am Fuß der Kolonne 61 g/h eines Gemisches aus 1,5 Gew.-% Phenol, 0,9 Gew.-% Methylphenylcarbonat und 97,6 Gew.-% Diphenylcarbonat. Das bedeutete eine Raumzeitausbeute für die Diphenylcarbonatbildung von 0,06 g/mlh, bezogen auf das Reaktorvolumen. 18,6 % des eingesetzten Methylphenylcarbonats reagierte mit Phenol unter Umesterung zum Diphenylcarbonat. Die Selektivität der Diphenylcarbonatbildung war >99,9 %, d.h., es konnten keine Nebenprodukte nachgewiesen werden.

Beispiel 16

(Fig. 1, Reaktor B, Reaktionsdestillation)

In der im Beispiel 15 beschriebenen Kolonnenapparatur wurde unter den dort angegebenen Bedingungen ein Gemisch aus 50 g/h Methylphenylcarbonat, 200 g/h Phenol und 3,25 g/h Octylstannonsäure umgesetzt. Nach dem Einstellen konstanter Bedingungen entnahm man am Kopf der Apparatur kontinuierlich 210 g/h eines Gemisches aus 1,0 Gew.-% Methanol, 5,4 Gew.-% Dimethylcarbonat und 93,6 Gew.-% Phenol und am Fuß der Kolonne 48 g/h eines Gemisches aus 9,4 Gew.-% Phenol, 1,9 Gew.-% Methylphenylcarbonat und 88,7 Gew.-% Diphenylcarbonat. Das bedeutete eine Raumzeitausheute für die Diphenylcarbonatbildung von $0,048\ g \cdot ml^{-1} \cdot h^{-1}$, bezogen auf das Reaktorvo-lumen. 20,5 % des eingesetzten Methylphenylcarbonats reagierte mit Phenol unter Umesterung zum Diphenylcarbonat. Die Selektivität der Diphenylcarbonatbildung war >99,9 %, d.h., es konnten keine Nebenprodukte nachgewiesen werden.

Beispiel 17

(Reaktor B, Reaktionsdestillation)

In einer wie im Beispiel 15 beschriebenen Kolonnenapparatur, bei der anstelle der 10 bödigen Siebbodenkolonne eine isotherm auf 183°C thermostatisierte, 10 bödige Glockenbodenkolonne von 72 cm Länge und 4,75 cm Innendurchmesser eingesetzt wurde, wurde unter den dort angegebenen Bedingungen ein Gemisch aus 150 g/h Methylphenylcarbonat, 350 g/h Phenol und 6,25 g/h Octylstannonsäure umgesetzt. Nach dem Einstellen konstanter Bedingungen entnahm man am Kopf der Apparatur kontinuierlich 365 g/h eines Gemisches aus 1,3 Gew.-% Methanol, 8,5 Gew.-% Dimethylcarbonat und 90,2 Gew.-% Phenol und am Fuß der Kolonne 135 g/h eines Gemisches aus 6,2 Gew.-% Phenol, 14,0 Gew.-% Methylphenylcarbonat und 79,8 Gew.-% Diphenylcarbonat. Das bedeutet eine Raumzeitausbeute für die Diphenylcarbonatbildung von $0,085\ g \cdot ml^{-1} \cdot h^{-1}$, bezogen auf das Reaktorvolumen. 16,6 % des eingesetzten Methylphenylcarbonats reagierte mit Phenol unter Umesterung zum Diphenylcarbonat. Die Selektivität der Diphenylcarbonatbildung war >99 %, d.h. es konnten keine Nebenprodukte nachgewiesen werden.

Aus den Beispielen erkennt man, daß durch die erfindungsgemäße, dem speziellen Umesterungsproblem angepaßte Fahrweise in der ersten Umesterungsstufe nach Gleichung 1 schon bei relativ niedrigen Temperaturen ohne Anwendung von Druck und großen Dialkylcarbonatüberschüssen erheblich höhere Raumzeitausbeuten der Arylcarbonatbildung und der Alkoholbildung von 0,02 bis 0,06 g/mlh, im Vergleich zum Stand der Technik (<0,02 g/mlh) erreicht werden, wobei gleichzeitig sehr hohe Selektivitäten >99,9 % erzielt werden können, d.h., es konnten keine Nebenprodukte >0,1 Gew.-% in den Produktströmen nachgewiesen werden. Die Umsetzung der Alkylarylcarbonate verläuft mit der erfindungsgemäßen Fahrweise in einer Reaktionsdestillationsapparatur fast quantitativ, wobei diese nicht nur durch eine Disproportionierungsreaktion gemäß Gleichung 3, sondern auch in erheblichem Maße durch Umesterung mit Phenol zum Diarylcarbonat umgesetzt werden.

**Patentansprüche**

1.  Verfahren zur Herstellung von Diarylcarbonaten der Formel

$$Ar^1 - O - CO - O - Ar^1$$

in der

$Ar^1$  nichtsubstituiertes Phenyl oder Naphthyl oder 1 bis 3fach substituiertes Phenyl, wobei die Substituenten gleich oder verschieden sind und zur Gruppe von $C_1$-$C_4$-Alkyl, $C_1$-$C_4$-Alkoxy und Halogen gehören, bedeutet,

durch Umesterung von 1 Mol von aromatischen Hydroxyverbindungen der Formel

$$Ar^1 - OH$$

worin $Ar^1$ die genannte Bedeutung hat,
mit 0,1 bis 10 Molen, bevorzugt mit 0,2 bis 5 Molen, besonders bevorzugt mit 0,5 bis 3 Molen Dialkylcarbonaten der Formel

$$R^1 - O - CO - O - R^1$$

in der

$R^1$  geradkettiges oder verzweigtes $C_1$-$C_6$-Alkyl, Cyclopentyl oder Cyclohexyl bedeutet,

in Gegenwart von an sich bekannten Umesterungskatalysatoren in für Umesterungen bekannten Kolonnenapparaten, dadurch gekennzeichnet, daß die Umesterungsreaktion in zwei hintereinander geschalteten Kolonnen in der Art durchgeführt wird, daß

a) in der ersten Kolonne die aromatische Hydroxyverbindung flüssig vom Kopf zum Sumpf geführt wird und das Dialkylcarbonat gasförmig diesem flüssigen Strom entgegengeführt wird, wobei die Temperatur im Bereich von 100 bis 300°C, bevorzugt 120 bis 250°C, besonders bevorzugt 150 bis 240°C und der Druck im Bereich von 0,5 bis 20 bar, bevorzugt 0,8 bis 15 bar, besonders bevorzugt 0,9 bis 10 bar, gehalten werden und das Dialkylcarbonat einen Anteil von 0 bis 5 Gew.-%, bevorzugt 0,1 bis 3 Gew.-%, besonders bevorzugt 0,2 bis 2 Gew.-%, des zugrundeliegenden Alkohols, bezogen auf das Gesamtgewicht von Dialkylcarbonat und Alkohol, enthält, daß

b) am Kopf der ersten Kolonne der dem Dialkylcarbonat zugrundeliegende und bei der Umesterung freiwerdende Alkohol gasförmig und am Sumpf der ersten Kolonne ein teilweise umgesetztes und im wesentlichen aus Alkylarylcarbonat bestehendes Umesterungsprodukt flüssig entnommen werden, daß

c) der Sumpfstrom der ersten Kolonne in den mittleren Teil einer zweiten Kolonne eingespeist wird, deren Temperatur im Bereich von 60 bis 320°C, bevorzugt 65 bis 305°C, besonders bevorzugt 65 bis 250°C und deren Druck im Bereich von 0,05 bis 10 bar, bevorzugt 0,1 bis 5 bar und besonders bevorzugt 0,2 bis 2 bar gehalten werden, wobei an diese zweite Kolonne in Abhängigkeit vom Siedepunkt des eingespeisten Sumpfstroms aus der ersten Kolonne ein solcher Temperatur-Gradient gelegt wird, daß das im eingespeisten Sumpfstrom enthaltene Alkylarylcarbonat aus dem mittleren Teil der zweiten Kolonne im wesentlichen weder zum Kopf noch zum Sumpf entweichen kann und daß

d) am Kopf der zweiten Kolonne ein Gemisch aus Dialkylcarbonat, dem zugrundeliegenden Alkohol und überschüssiger aromatischer Hydroxyverbindung und am Sumpf der zweiten Kolonne Dialkylcarbonat, das Alkylarylcarbonat und aromatische Hydroxyverbindung enthalten kann, entnommen werden.

2. Verfahren nach Anspruch 1, dadurch gekennzeichnet, daß aromatische Hydroxyverbindungen der Formel

$$Ar^{11}\text{-OH},$$

eingesetzt werden, in der

Ar$^{11}$      Phenyl oder einfach durch $C_1$-$C_4$-Alkyl, $C_1$-$C_4$-Alkoxy oder Chlor substituiertes Phenyl bedeutet,

und daß bevorzugt nicht substituiertes Phenol eingesetzt wird.

3. Verfahren nach Anspruch 1, dadurch gekennzeichnet, daß als Dialkylcarbonate das Dimethyl- oder Diethylcarbonat, bevorzugt das Dimethylcarbonat eingesetzt wird (werden).

4. Verfahren nach Anspruch 1, dadurch gekennzeichnet, daß im Verfahrensabschnitt gemäß c) im mittleren Teil der zweiten Kolonne ein Phenolüberschuß von 2 bis 20 Mol, bevorzugt von 3-15 Mol, besonders bevorzugt von 3 bis 10 Mol pro Mol Alkylarylcarbonat eingestellt wird.

5. Verfahren nach Anspruch 1, dadurch gekennzeichnet, daß die erste Kolonne mit Füllkörpern oder Einbauten ausgerüstet ist, eine theoretische Bodenzahl von 3 bis 50, bevorzugt 3 bis 30, besonders bevorzugt 5 bis 20, hat und ihr Flüssigkeits-Hold-up 1 bis 80 %, bevorzugt 10 bis 75 %, besonders bevorzugt 15 bis 70 %, ganz besonders bevorzugt 15-50 % des Kolonneninnenvolumens beträgt.

6. Verfahren nach Anspruch 1, dadurch gekennzeichnet, daß die zweite Kolonne mit Füllkörpern oder Einbauten ausgerüstet ist, bevorzugt im mittleren Teil mit Einbauten und in dem darunter und in dem darüberliegenden Teil mit Füllkörpern ausgerüstet ist.

7. Verfahren nach Anspruch 6, dadurch gekennzeichnet, daß der mittlere, mit Einbauten ausgerüstete Teil der zweiten Kolonne eine theoretische Bodenzahl von 2 bis 50, bevorzugt 2 bis 25, besonders bevorzugt 2 bis 15, und ein Flüssigkeits-Hold-up von 5 bis 80 %, bevorzugt 10 bis 75 %, besonders bevorzugt 15 bis 70 %, ganz besonders bevorzugt 15 bis 50 % des Innenvolumens der Einbauten hat.

8. Verfahren nach Anspruch 1, dadurch gekennzeichnet, daß dem Sumpf der zweiten Kolonne eine weitere Destillationskolonne zur Reindarstellung des Diarylcarbonats nachgeschaltet wird.

## Claims

1. Process for the preparation of diaryl carbonates of the formula

$$Ar^1 \text{ - O - CO - O - } Ar^1$$

in which

Ar$^1$      denotes unsubstituted phenyl or naphthyl or monosubstituted to trisubstituted phenyl, the substituents being identical or different and belonging to the group consisting of $C_1$-$C_4$-alkyl, $C_1$-$C_4$-alkoxy and halogen,

by transesterification of 1 mol of aromatic hydroxyl compounds of the formula

$$Ar^1 \text{ - OH}$$

in which Ar$^1$ has the meaning mentioned,
with 0.1 to 10 mol, preferably 0.2 to 5 mol, particularly preferably 0.5 to 3 mol, of dialkyl carbonates of the formula

$$R^1 \text{ - O - CO - O - } R^1$$

in which

$R^1$ denotes straight-chain or branched $C_1$-$C_6$-alkyl, cyclopentyl or cyclohexyl,

in the presence of transesterification catalysts known per se in column apparatuses known for transesterifications, characterised in that the transesterification reaction is carried out in two columns connected one after the other in such a way that

a) in the first column, the aromatic hydroxyl compound is conducted in the liquid state from head to bottom and the dialkyl carbonate is conducted in the gaseous state against this liquid stream, the temperature being kept in the range from 100 to 300°C, preferably 120 to 250°C, particularly preferably 150 to 240°C and the pressure being kept in the range from 0.5 to 20 bar, preferably 0.8 to 15 bar, particularly preferably 0.9 to 10 bar, and the dialkyl carbonate containing a portion from 0 to 5 % by weight, preferably 0.1 to 3 % by weight, particularly preferably 0.2 to 2 % by weight, of the underlying alcohol, based on the overall weight of dialkyl carbonate and alcohol, that

b) at the head of the first column, the alcohol underlying the dialkyl carbonate and liberated in the transesterification is withdrawn in the gaseous state and at the bottom of the first column, a partly reacted transesterification product essentially composed of alkyl aryl carbonate is withdrawn in the liquid state, that

c) the bottom stream of the first column is fed into the central part of a second column, the temperature of which is kept in the range from 60 to 320°C, preferably 65 to 305°C, particularly preferably 65 to 250°C and the pressure of which is kept in the range from 0.05 to 10 bar, preferably 0.1 to 5 bar and particularly preferably 0.2 to 2 bar, depending on the boiling point of the bottom stream fed in from the first column, a temperature gradient being applied to this second column in such a way that the alkyl aryl carbonate contained in the bottom stream fed in can escape from the central part of the second column essentially neither to the head nor to the bottom and that

d) at the head of the second column, a mixture of dialkyl carbonate, the underlying alcohol and excess aromatic hydroxyl compound and at the bottom of the second column, diaryl carbonate, which can contain alkyl aryl carbonate and aromatic hydroxyl compound, are withdrawn.

2. Process according to Claim 1, characterised in that aromatic hydroxyl compounds of the formula

$$Ar^{11}\text{-OH,}$$

are used, in which

$Ar^{11}$ denotes phenyl or phenyl monosubstituted by $C_1$-$C_4$-alkyl, $C_1$-$C_4$-alkoxy or chlorine,

and that unsubstituted phenol is preferably used.

3. Process according to Claim 1, characterised in that dimethyl carbonate or diethyl carbonate, preferably dimethyl carbonate, is (are) used as the dialkyl carbonates.

4. Process according to Claim 1, characterised in that in the process section according to c), in the central part of the second column, a phenol excess of 2 to 20 mol, preferably 3-15 mol, particularly preferably 3 to 10 mol per mole of alkyl aryl carbonate is established.

5. Process according to Claim 1, characterised in that the first column is equipped with packings or internals, has a theoretical plate number from 3 to 50, preferably 3 to 30, particularly preferably 5 to 20, and its liquid holdup is 1 to 80 %, preferably 10 to 75 %, particularly preferably 15 to 70 %, very particularly preferably 15-50 % of the column internal volume.

6. Process according to Claim 1, characterised in that the second column is equipped with packings or internals, preferably in the central part with internals and is equipped with packings in the part below and the part above.

7. Process according to Claim 6, characterised in that the central part of the second column which is equipped with internals has a theoretical plate number from 2 to 50, preferably 2 to 25, particularly preferably 2 to 15, and a liquid holdup from 5 to 80 %, preferably 10 to 75 %, particularly preferably 15 to 70 %, very particularly preferably 15 to 50 % of the internal volume of the internals.

8. Process according to Claim 1, characterised in that a further distillation column for isolation of the diaryl carbonate is connected downstream of the bottom of the second column.

**Revendications**

1. Procédé pour la préparation de carbonates de diaryles de la formule

$$Ar^1\text{-}O\text{-}CO\text{-}O\text{-}Ar^1$$

dans laquelle $Ar^1$ est un groupe phényle ou naphthyle non substitué ou un groupe phényle substitué de 1 à 3 fois, les substituants étant identiques ou différents et appartenant au groupe constitué de groupes alkyle en $C_1$-$C_4$, alcoxy en $C_1$-$C_4$ et d'halogènes,
par trans-estérification de 1 mol de composé aromatique hydroxy de la formule

$$Ar^1\text{-}OH$$

où $Ar^1$ a la signification citée,
avec de 0,1 à 10 mol, de préférence avec de 0,2 à 5 mol, encore mieux avec de 0,5 à 3 mol de carbonates de dialkyles de la formule

$$R^1\text{-}O\text{-}CO\text{-}O\text{-}R^1$$

dans laquelle $R^1$ est un groupe alkyle en $C_1$-$C_6$, linéaire ou ramifié, cyclopentyle ou cyclohexyle en présence de catalyseurs de trans-estérification connus en eux-mêmes dans des appareils de type colonne connus pour la trans-estérification, caractérisé en ce que la réaction de trans-estérification est réalisée dans deux colonnes raccordées l'une derrière l'autre de telle sorte que

a) le composé aromatique hydroxy est introduit sous forme liquide dans la première colonne de la tête vers la queue et que le carbonate de dialkyle est introduit sous forme gazeuse à contre-courant de ce courant liquide, la température étant maintenue dans un domaine de 100 à 300°C, de préférence de 120 à 250°C, encore mieux de 150 à 240°C et la pression dans un domaine de 0,5 à 20 bar, de préférence de 0,8 à 15 bar, encore mieux de 0,9 à 10 bar et le carbonate de dialkyle contient une part de 0 à 5 % en poids, de préférence de 0,1 à 3 % en poids, encore mieux de 0,2 à 2 % en poids de l'alcool de base, rapportés au poids total du carbonate de dialkyle et de l'alcool, que
b) l'alcool à la base du carbonate de dialkyle et libéré lors de la trans-estérification est prélevé sous forme gazeuse en tête de la première colonne et un produit de trans-estérification ayant partiellement réagi et essentiellement constitué de carbonate d'alkylaryle est prélevé sous forme liquide en queue de la première colonne, que
c) le courant de queue de la première colonne est introduit dans la partie médiane d'une seconde colonne, dont la température est maintenue dans un domaine de 60 à 320°C, de préférence de 65 à 305°C, encore mieux de 65 à 250°C et dont la pression est maintenue dans un domaine de 0,05 à 10 bar, de préférence de 0,1 à 5 bar et encore mieux de 0,2 à 2 bar, un gradient de température étant appliqué à cette seconde colonne selon le point d'ébullition du courant de queue introduit à partir de la première colonne, de telle sorte que le carbonate d'alkylaryle contenu dans le courant de queue introduit peut se dégager de la partie médiane de la seconde colonne en substance soit vers la tête, soit vers la queue et que
d) un mélange de carbonate de dialkyle, de l'alcool de base et de composé aromatique hydroxy en excès est prélevé en tête de la seconde colonne et que du carbonate de diaryle, qui peut contenir du carbonate d'alkylaryle et du composé aromatique hydroxy, est prélevé en queue de la seconde colonne.

2. Procédé selon la revendication 1, caractérisé en ce que l'on utilise des composés aromatiques hydroxy de la formule

$$Ar^{11}\text{-}OH,$$

dans laquelle $Ar^{11}$ est un groupe phényle ou un groupe phényle substitué une fois par un groupe alkyle en $C_1$-$C_4$, alcoxy en $C_1$-$C_4$ ou par le chlore
et en ce que l'on utilise de préférence du phénol non substitué.

3. Procédé selon la revendication 1, caractérisé en ce qu'on utilise comme carbonates de dialkyles le carbonate de diméthyle ou de diéthyle, de préférence le carbonate de diméthyle.

4. Procédé selon la revendication 1, caractérisé en ce que l'on règle dans le tronçon du procédé selon c) dans la partie médiane de la seconde colonne un excès de phénol de 2 à 20 mol, de préférence de 3-15 mol, encore mieux de 3 à 10 mol par mole de carbonate d'alkylaryle.

5. Procédé selon la revendication 1, caractérisé en ce que la première colonne est munie de corps de remplissage ou de chicanes, a un nombre théorique de plateaux de 3 à 50, de préférence de 3 à 30, encore mieux de 5 à 20 et son hold-up de liquide est de 1 à 80 %, de préférence de 10 à 75 %, encore mieux de 15 à 70 %, bien mieux encore de 15-50 % du volume interne de la colonne.

6. Procédé selon la revendication 1, caractérisé en ce que la seconde colonne est munie de corps de remplissage ou de chicanes, de préférence de plateaux dans la partie médiane et de corps de remplissage dans la partie se trouvant au-dessous et au-dessus.

7. Procédé selon la revendication 6, caractérisé en ce que la partie médiane de la seconde colonne munie de chicanes a un nombre théorique de plateaux de 2 à 50, de préférence de 2 à 25, encore mieux de 2 à 15 et un hold-up de liquide de 5 à 80 %, de préférence de 10 à 75 %, encore mieux de 15 à 70 %, bien mieux encore de 15 à 50 % du volume interne des chicanes.

8. Procédé selon la revendication 1, caractérisé en ce qu'une autre colonne de distillation est raccordée en aval de la queue de la seconde colonne pour une purification du carbonate de diaryle.

Fig.1

Fig.2